# EUROPÄISCHE PATENTANMELDUNG

(11) **EP 2 998 290 A1**
(43) Veröffentlichungstag der Anmeldung: **23.03.2016**
(21) Anmeldenummer: 14184965.3
(22) Anmeldetag: 16.09.2014
(51) Int. Cl.: C07C 253/10, C07C 253/30, C07C 255/04, C07C 255/07

(54) **Verfahren zur kontinuierlichen Herstellung von Adipodinitril**

(71) Anmelder: BASF SE, 67056 Ludwigshafen (DE)
(72) Erfinder: FISCHER, Rolf-Hartmuth, 69121 Heidelberg (DE); BAUMANN, Robert, 68529 Mannheim (DE); WLOKA, Veronika, 67133 Maxdorf (DE); LUYKEN, Hermann, 67069 Ludwigshafen (DE)
(74) Vertreter: Féaux de Lacroix, Stefan

(57) **Zusammenfassung**

Beschrieben wird ein Verfahren zur kontinuierlichen Herstellung von Adipodinitril durch Hydrocyanierung von 3-Pentennnitril, bei dem man
a) 3-Pentennitril hydrocyaniert unter Erhalt eines Reaktionsaustrags, der Adipodinitril enthält,
b) in einer Aufarbeitung 1 aus dem Reaktionsaustrag des Reaktors R1 in einer ersten Destillationsvorrichtung als Kopfprodukt ein Gemisch enthaltend cis-2-Methyl-2-butennitril und cis-2-Pentennitril abtrennt,
c) das Gemisch enthaltend cis-2-Methyl-2-butennitril und cis-2-Pentennitril aus Schritt b) in einem Reaktor R2 in Gegenwart von Aluminiumoxid als Katalysator kontinuierlich zu einem 3-Pentennitril enthaltenden Produktgemisch isomerisiert,
d) aus dem Reaktionsaustrag des Reaktors R2 in einer Aufarbeitung 2 in einer Destillationsvorrichtung cis-2-Methyl-2-butennitril als Kopfprodukt abtrennt und ausschleust.

## Beschreibung

Die Erfindung betrifft Verfahren zur kontinuierlichen Herstellung von Adipodinitril durch Hydrocyanierung von 3-Pentennitril, wobei cis-2-Pentennitril an einem Aluminiumoxid enthaltenden Katalysator zu 3-Pentennitril isomerisiert wird.

Adipodinitril wird großtechnisch nach drei unterschiedlichen Verfahren hergestellt. Im Einzelnen erfolgt dies durch i) Umsetzung von Adipinsäure mit Ammoniak, ii) durch Dimerisierung von Acrylnitril oder iii) durch Hydrocyanierung von Butadien mit Blausäure.

Weltweit wird Adipodinitril ganz überwiegend durch Hydrocyanierung von Butadien produziert.

Im ersten Hydrocyanierungsschritt wird Butadien mit Blausäure in Gegenwart von Nickel(0)-Phosphorligand-Komplexverbindungen zu Gemischen umgesetzt, die überwiegend 3-Pentennitrli und 2-Methyl-3-butennitril enthalten. 3-Pentennitril und 2-Methyl-3-butennitriil werden destillativ getrennt. 2-Methyl-3-butennitril wird zu 3-Pentennitril isomerisiert.

Im zweiten Hydrocyanierungsschritt wird 3-Pentennitril mit Blausäure in Gegenwart von Nickel(0)-Phosphorligand-Komplexen und zusätzlich einer Lewis-Säure zu Adipodinitril hydrocyaniert.

Die Herstellung von Adipodinitril durch Hydrocyanierung von 3-Pentennitril ist beispielsweise in WO 2005/073173 beschrieben.

Bei der Hydrocyanierung von 3-Pentennitril entstehen cis-2-Pentennitril und trans-2-Pentennitril. Beide Isomeren lassen sich in Gegenwart der Nickel(0)-Phosphorligand-Komplexe nicht oder nur in Gegenwart spezieller Ni(0)-Phosphorligand-Komplexe (US 2008/15379 A1) mit Blausäure zu Adipodinitril hydrocyanieren. Trotzdem stellen cis-und trans-2-Pentennitril potentielle Wertprodukte dar.

Die nachstehende Tabelle zeigt, dass cis-2-Pentennitril gegenüber den restlichen vier linearen Pentennitril-Isomeren einen deutlich niedrigeren Siedepunkt besitzt und sich daher mit vertretbarem Energieaufwand destillativ von den restlichen linearen Pentennitrilen abtrennen lässt.

**C₅-Nitril-Siedepunkte**

| C₅-Nitrile | Siedepunkt (1013 mbar) |
|---|---|
| cis-2-Methyl-2-butennitril (= Z) | 120-123°C |
| 2-Methyl-3-butennitril | 124°C |
| cis-2-Pentennitril (cis-2-PN) | 127-128°C |
| trans-2-Methyl-2-butennitril (= E) | 138°C |
| trans-2-Pentennitril (trans-2-PN) | 143-145°C |
| cis-3-Pentennitril (cis-3-PN) | 142-144°C |
| trans-3-Pentennitril (trans-3-PN) | 142-146°C |
| 4-Pentennitril (4-PN) | 140-147°C |

Es ist weiterhin bekannt, cis-2-Pentennitril in Gegenwart von stark basischen Verbindungen zu 3-Pentennitril enthaltenden Pentennitril-Gemischen zu isomerisieren, siehe Prochazka et al., Collection of Czechoslovak Chemical Communications 1970, Band 35, Seiten 1224 bis 1234. Gearbeitet wurde bei maximal 60°C und Normaldruck mit Kalium-tert.butylat als Base in tert.Butanol als Lösungsmittel. Bei dieser Temperatur tritt offenbar keine Michael-Addition des Alkohols an das Acrylnitril-Derivat cis-2-Pentennitril ("3-Ethylacrylnitril") auf.

Bei 60°C liegt das thermodynamische Gleichgewicht der linearen Pentennitrile bei 39.6% cis-2-Pentennitril, 33,4% trans-2-Pentennitril, 8,5% cis-3-Pentennitril und 18,5% trans-3-Pentennitril, siehe Tabelle 6 in der oben genannten Literaturstelle von Prochazka. Nachteilig für ein technisches Verfahren ist die niedrige Temperatur, die zu langen Isomerisierungszeiten führt, die Verwendung eines Lösungsmittels und die Notwendigkeit, den homogenen Katalysator zurückzuführen.

Die Isomerisierung von 2-Pentennitril zur Bildung von trans-3-Pentennitril in Gegenwart von Aluminiumoxid als Katalysator ist an sich aus der WO 2004/094364 bekannt. Es ist ferner beschrieben, dass das zur Isomerisierung eingesetzte cis-2-Pentennitril aus der Hydrocyanierung von 3-Pentennitril stammt. Gemäß der Beispiele wird die Isomerisierung an nicht näher beschriebenem Aluminiumoxid-Pulver bei einer Temperatur von 126 bis 144°C diskontinuierlich durchgeführt. Für das Aluminiumoxid ist lediglich angegeben, dass es vorzugsweise eine BET-Oberfläche von 70 bis 350 m²/g aufweisen soll.

Die vier diskontinuierlichen erfindungsgemäßen Ausführungsbeispiele wurden bei 126 bis 144°C durchgeführt (Tabelle in WO 2004/094364). Sie zeigen, dass die Geschwindigkeit der Isomerisierung bis zur Einstellung des Isomerisierungs-Gleichgewichts mit zunehmender BET-Oberfläche des Al₂O₃ ansteigt. In gleicher Richtung nimmt aber auch die Oligomerenbildung zu. Sie beträgt bei einer BET-Oberfläche von 106 m²/g 0,17%, von 250 m²/g 0,84% und 349 m²/g 1,4%.

Vergleichsbeispiel 1 zeigt dagegen, dass eine BET-Oberfläche von 31,5 m²/g innerhalb von 7 Stunden bei 126 bis 144°C nicht zur vollständigen Einstellung des Gleichgewichts führt. Es werden nur 3,7% trans-3-+cis-3-Pentennitril gebildet.

Angaben zur kontinuierlichen Isomerisierung von cis-2-Pentennitril in der Flüssigphase sind in WO 2004/094364A1 nicht enthalten.

Die Isomerisierung von cis-2-Pentennitril zu 3-Pentennitril in einer Reaktivdestillation ist in WO 2005/073177 beschrieben. Als Katalysator werden Stränge aus Al₂O₃ verwendet. Die Sumpftemperatur beträgt gemäß dem Beispiel 149°C. Der Al₂O₃-Katalysator wird in Strangform eingesetzt. Er wird ansonsten nicht näher erläutert.

U.S. 3 526 654 offenbart die Isomerisierung von cis-2-Pentennitril zu 3-Pentennitril in Gegenwart von Al₂O₃ (2 Beispiele), Silicagel (2 Beispiele) oder Natrium-Calcium-Silikat (ein Beispiel) in der Flüssig-oder Gasphase bei Temperaturen von 25 bis 500°C an suspendierten oder fest angeordneten Katalysatoren:

In Beispiel 1 wird die Isomerisierung von cis-2-Pentennitril bei 200°C in der Gasphase in Gegenwart von Alcoa F--1 Al₂O₃ und Nickel-Formkörpern kontinuierlich durchgeführt. Der Versuch wurde nur vier Stunden lang betrieben. Nach dieser Reaktionszeit enthielt das Pentennitril-Isomeren-Gemisch 43,3%% cis-2-Pentennitril, 34,9% trans-2-Pentennitril, 5,9% cis-3-Pentennitril und 17.6% trans-3-Pentennitril.

Nachteilig an der Gasphasen-Isomerisierung ist, dass mit niedrigen Katalysator-Standzeiten zu rechnen ist. Bei Einsatz von cis-2-Pentennitril, einem Acrylnitril-Derivat ist bei 200°C mit Oligomeren- und/oder Polymerenbildung, verbunden mit einer Desaktivierung des Katalysators, zu rechnen.

In Beispiel 3 wird die cis-2-Pentennitril-Isomerisierung diskontinuierlich bei Raumtemperatur in der Flüssigphase in Gegenwart von eta-Al₂O₃ durchgeführt. Nach sechs Monaten beträgt der cis-2-Pentennitril-Umsatz 40%. Das Pentennitril-Isomeren-Gemisch enthält 59,1% cis-2-Pentennitril, 24.1% trans-2-Pentennitril, 1.4% cis-3-Pentennitril und 15,4% trans-3-Pentennitril.

Eine Reaktionszeit von sechs Monaten ist für ein technisches Verfahren unwirtschaftlich. Der Fachmann muss aus der langen Reaktionszeit schließen, dass man die Reaktionstemperatur beim Arbeiten in der Flüssigphase nicht erhöhen sollte.

Produktionsanlagen zur Herstellung von Adipodinitril (ADN) durch Hydrocyanierung von Butadien und anschließend von 3-Pentennitril besitzen Produktionskapazitäten von 100 000 Jahrestonnen und mehr. Daher kommen zur Herstellung derartiger ADN-Mengen bevorzugt kontinuierliche Verfahren in Frage.

Die Aufgabe der vorliegenden Erfindung besteht daher darin, den Verfahrensschritt der Isomerisierung von cis-2-Pentennitril kontinuierlich durchzuführen und dieses Verfahren zur kontinuierlichen Isomerisierung in ein kontinuierliches Verfahren zur Herstellung von Adipodinitril aus Butadien, Blausäure und Wasserstoff zu integrieren, ohne dass sich störende Nebenkomponenten aufpegeln.

Vorzugsweise oder alternativ soll die kontinuierliche Isomerisierung mit möglichst wenigen Apparaten verwirklicht werden und besonders bevorzugt die Aufarbeitung der Reaktionsprodukte apparativ integrieren.

Erforderlich ist eine hohe Katalysator-Standzeit, das heißt, es soll keine wesentliche Deaktivierung des Isomerisierungs-Katalysators, weiterhin keine störende Belegung des Katalysators oder von Apparateteilen durch Oligomere und/oder Polymere erfolgen. Dabei sollen bei der Isomerisierung eine hohe Produktausbeute und Raum-Zeitausbeute an hydrocyanierbaren Zwischenprodukten und möglichst geringe Mengen an Oligomeren und Polymeren erzeugt werden. Eine Aufpegelung der Nebenprodukte cis- und trans 2-Methyl-2-butennitril sollte verhindert werden.

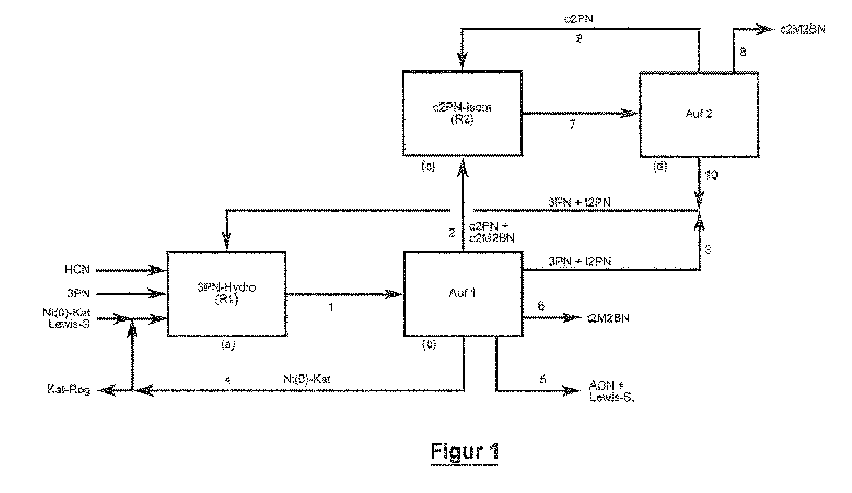

Die Aufgabe wird erfindungsgemäß gelöst durch ein - in Figur 1 schematisch dargestelltes - Verfahren zur kontinuierlichen Herstellung von Adipodinitril durch Hydrocyanierung von 3-Pentennnitril, bei dem man
a) 3-Pentennitril oder eine Mischung enthaltend 3-Pentennitril in einem Reaktor R1 mit Blausäure in Gegenwart von mindestens einem Nickel(0)-Komplex als Katalysator, freiem Liganden und mindestens einer Lewis-Säure hydrocyaniert unter Erhalt eines Reaktionsaustrags, der Adipodinitril, 2-Methylglutaronitril, den Nickel(0)-Komplex, freien Liganden, Lewis-Säure, Katalysator-Abbauprodukte, nicht umgesetztes 3-Pentennitril und als Nebenkomponenten cis- und trans-2-Methyl-2-butennitril sowie cis- und trans-2-Pentennitril enthält,
b) in einer Aufarbeitung 1 aus dem Reaktionsaustrag des Reaktors R1 in einer ersten Destillationsvorrichtung als Kopfprodukt ein Gemisch enthaltend cis-2-Methyl-2-butennitril und cis-2-Pentennitril, als Sumpfprodukt ein Gemisch enthaltend Adipodinitril, Nickel(0)-Komplex, freien Liganden, mindestens eine Lewis-Säure und Katalyator-Abbauprodukte und an einem Seitenabzug ein Gemisch enthaltend trans-2-Methyl-2-butennitril, trans-2-Pentennitril und 3-Pentennitril abtrennt, das Seitenabzugsprodukt in einer zweiten Destillationsvorrichtung so auftrennt, dass als Kopfprodukt trans-2-Methyl-2-butennitril abgetrennt und ausgeschleust wird und als Sumpfprodukt 3-Pentennitril und trans-2-Pentennitril erhalten und in die 3-Pentennitril-Hydrocyanierung in Schritt a) zurückgeführt werden,
c) das Gemisch enthaltend cis-2-Methyl-2-butennitril und cis-2-Pentennitril aus Schritt b) in einem Reaktor R2 in Gegenwart von Aluminiumoxid als Katalysator kontinuierlich zu einem 3-Pentennitril enthaltenden Produktgemisch isomerisiert, wobei die Isomerisierung bei Temperaturen von 120 bis 220°C und Drücken von 1 bis 15 bar in der Flüssigphase erfolgt, das Aluminiumoxid eine BET-Oberfläche von 50 bis 450 m²/g und einen pH-Wert von 4 bis 10,5 besitzt,
d) aus dem Reaktionsaustrag des Reaktors R2 in einer Aufarbeitung 2 in einer Destillationsvorrichtung cis-2-Methyl-2-butennitril als Kopfprodukt abtrennt und ausschleust, nicht umgesetztes cis-2-Pentennitril aus einem Seitenabzug abtrennt und in den Reaktor R2 in Schritt c) und das 3-Pentennitril enthaltende Sumpfprodukt in die 3-Pentennitril-Hydrocyanierung in Schritt a) zurückführt.

Die Aufgabe wird zudem gelöst durch ein Verfahren zur kontinuierlichen Herstellung von Adipodinitril durch Hydrocyanierung von 3-Pentennnitril, bei dem man
a) 3-Pentennitril oder eine Mischung enthaltend 3-Pentennitril in einem Reaktor R1 mit Blausäure in Gegenwart von mindestens einem Nickel(0)-Komplex als Katalysator, freiem Liganden und mindestens einer Lewis-Säure hydrocyaniert unter Erhalt eines Reaktionsaustrags, der Adipodinitril, 2-Methylglutaronitril, den Nickel(0)-Komplex, freien Liganden, Lewis-Säure, Katalysator-Abbauprodukte, nicht umgesetztes 3-Pentennitril und als Nebenkomponenten cis- und trans-2-Methyl-2-butennitril sowie cis- und trans-2-Pentennitril enthält,
b) in einer Aufarbeitung 1 aus dem Reaktionsaustrag des Reaktors R1 in einer ersten Destillationsvorrichtung als Kopfprodukt nur cis-2-Methyl-2-butennitril, als Sumpfprodukt alle restlichen Verbindungen abtrennt und in einer zweiten Destillationsvorrichtung das Sumpfprodukt der ersten Destillationsvorrichtung so auftrennt, dass als Kopfprodukt cis-2-Pentennitril, über einen Seitenabzug 3-Pentennitril, trans-2-Pentennitril und trans-2-Methyl-2-butennitril und als Sumpfprodukt Roh-Adipodinitril, Nickel-(0)-Komplex, freier Ligand, die mindestens eine Lewis-Säure und Katalysatorabbauprodukte erhalten werden, und das Seitenabzugsprodukt der zweiten Destillationsvorrichtung in einer dritten Destillationsvorrichtung so destilliert wird, dass trans-2-Methyl-2-butennitril als Kopfprodukt ausgeschleust wird und als Sumpfprodukt ein Gemisch enthaltend trans-2-Pentennitril und 3-Pentennitril abgetrennt und in die 3-Pentennitril-Hydrocyanierung in Schritt a) zurückgeführt wird,
c) das cis-2-Pentennitril aus Schritt b) in einem Reaktor R2 in Gegenwart von Aluminiumoxid als Katalysator kontinuierlich zu einem 3-Pentennitril enthaltenden Produktgemisch isomerisiert, wobei die Isomerisierung bei Temperaturen von 120 bis 220°C und Drücken von 1 bis 15 bar in der Flüssigphase erfolgt, das Aluminiumoxid eine BET-Oberfläche von 50 bis 450 m²/g und einen pH-Wert von 4 bis 10,5 besitzt,
d) aus dem Reaktionsaustrag des Reaktors R2 in einer Aufarbeitung 2 in einer Destillationsvorrichtung nicht umgesetztes cis-2-Pentennitril als Kopfprodukt abtrennt und in den Reaktor R2 in Schritt c) und das 3-Pentennitril enthaltende Sumpfprodukt in die 3-Pentennitril-Hydrocyanierung in Schritt a) zurückführt.

Die Aufgabe wird ferner gelöst durch ein Verfahren zur kontinuierlichen Herstellung von Adipodinitril durch Hydrocyanierung von 3-Pentennnitril, bei dem man
a) 3-Pentennitril oder eine Mischung enthaltend 3-Pentennitril in einem Reaktor R1 mit Blausäure in Gegenwart von mindestens einem Nickel(0)-Komplex als Katalysator, freiem Liganden und mindestens einer Lewis-Säure hydrocyaniert unter Erhalt eines Reaktionsaustrags, der Adipodinitril, 2-Methylglutaronitril, den Nickel(0)-Komplex, freien Liganden, Lewis-Säure, Katalysator-Abbauprodukte, nicht umgesetztes 3-Pentennitril und als Nebenkomponenten cis- und trans-2-Methyl-2-butennitril sowie cis- und trans-2-Pentennitril enthält,
b) in einer Aufarbeitung 1 aus dem Reaktionsaustrag des Reaktors R1 in einer ersten Destillationsvorrichtung als Kopfprodukt ein Gemisch enthaltend cis-2-Methyl-2-butennitril und cis-2-Pentennitril, als Sumpfprodukt ein Gemisch enthaltend Adipodinitril, Nickel(0)-Komplex, freien Liganden, mindestens eine Lewis-Säure und Katalyator-Abbauprodukte und an einem Seitenabzug ein Gemisch enthaltend trans-2-Methyl-2-butennitril, trans-2-Pentennitril und 3-Pentennitril abtrennt, das Seitenabzugsprodukt in einer zweiten Destillationsvorrichtung so auftrennt, dass als Kopfprodukt trans-2-Methyl-2-butennitril abgetrennt und ausgeschleust wird und als Sumpfprodukt 3-Pentennitril und trans-2-Pentennitril erhalten und in die 3-Pentennitril-Hydrocyanierung in Schritt a) zurückgeführt werden,
c) das Gemisch enthaltend cis-2-Methyl-2-butennitril und cis-2-Pentennitril aus Schritt b) in einer Reaktivdestillationskolonne R2 in Gegenwart von Aluminiumoxid als Katalysator in einer Reaktionszone kontinuierlich zu einem 3-Pentennitril enthaltenden Produktgemisch isomerisiert, wobei die Isomerisierung bei Temperaturen von 120 bis 220°C und Drücken von 1 bis 15 bar in der Flüssigphase erfolgt, das Aluminiumoxid eine BET-Oberfläche von 50 bis 450 m²/g und einen pH-Wert von 4 bis 10,5 besitzt, und cis-2-Methyl-2-butennitril als Kopfprodukt abtrennt und ausschleust, nicht umgesetztes cis-2-Pentennitril aus einem Seitenabzug abtrennt und in den Reaktor R2 in Schritt c) und das 3-Pentennitril enthaltende Sumpfprodukt in die 3-Pentennitril-Hydrocyanierung in Schritt a) zurückführt.

Die Aufgabe wird des Weiteren gelöst durch ein Verfahren zur kontinuierlichen Herstellung von Adipodinitril durch Hydrocyanierung von 3-Pentennnitril, bei dem man
a) 3-Pentennitril oder eine Mischung enthaltend 3-Pentennitril in einem Reaktor R1 mit Blausäure in Gegenwart von mindestens einem Nickel(0)-Komplex als Katalysator, freiem Liganden und mindestens einer Lewis-Säure hydrocyaniert unter Erhalt eines Reaktionsaustrags, der Adipodinitril, 2-Methylglutaronitril, den Nickel(0)-Komplex, freien Liganden, Lewis-Säure, Katalysator-Abbauprodukte, nicht umgesetztes 3-Pentennitril und als Nebenkomponenten cis- und trans-2-Methyl-2-butennitril sowie cis- und trans-2-Pentennitril enthält,
b) in einer Aufarbeitung 1 aus dem Reaktionsaustrag des Reaktors R1 in einer ersten Destillationsvorrichtung als Kopfprodukt nur cis-2-Methyl-2-butennitril, als Sumpfprodukt alle restlichen Verbindungen abtrennt und in einer zweiten Destillationsvorrichtung das Sumpfprodukt der ersten Destillationsvorrichtung so auftrennt, dass als Kopfprodukt cis-2-Pentennitril, über einen Seitenabzug 3-Pentennitril, trans-2-Pentennitril und trans-2-Methyl-2-butennitril und als Sumpfprodukt Roh-Adipodinitril, Nickel-(0)-Komplex, freier Ligand, die mindestens eine Lewis-Säure und Katalysatorabbauprodukte erhalten werden, und das Seitenabzugsprodukt der zweiten Destillationsvorrichtung in einer dritten Destillationsvorrichtung so destilliert wird, dass trans-2-Methyl-2-butennitril als Kopfprodukt ausgeschleust wird und als Sumpfprodukt ein Gemisch enthaltend trans-2-Pentennitril und 3-Pentennitril abgetrennt und in die 3-Pentennitril-Hydrocyanierung in Schritt a) zurückgeführt wird,
c) cis-2-Pentennitril aus Schritt b) in einer Reaktivdestillationskolonne R2 in Gegenwart von Aluminiumoxid als Katalysator in einer Reaktionszone kontinuierlich zu einem 3-Pentennitril enthaltenden Produktgemisch isomerisiert, wobei die Isomerisierung bei Temperaturen von 120 bis 220°C und Drücken von 1 bis 15 bar in der Flüssigphase erfolgt, das Aluminiumoxid eine BET-Oberfläche von 50 bis 450 m²/g und einen pH-Wert von 4 bis 10,5 besitzt, und nicht umgesetztes cis-2-Pentennitril als Kopfprodukt abtrennt und in den Reaktor R2 in Schritt c) und das 3-Pentennitril enthaltende Sumpfprodukt in die 3-Pentennitril-Hydrocyanierung in Schritt a) zurückführt.
Gemäß einer Ausführungsform der Erfindung wird in der Aufarbeitung 1 in der ersten Destillationsvorrichtung ein Kopfprodukt, enthaltend cis-2-Methyl-2-butennitril und cis-3-Pentennitril, ausgetragen und in die nachfolgende Isomerisierung überführt. Alternativ kann in der ersten Destillationsvorrichtung als Kopfprodukt nur cis-2-Methyl-2-butennitril ausgetragen und in die Isomerisierung überführt werden.

Zudem ist es bei beiden Ausführungsformen des erfindungsgemäßen Verfahrens möglich, die Isomerisierung in Schritt c) mit der Aufarbeitung 2 zu kombinieren. Dabei wird die Isomerisierung in einer Reaktivdestillationskolonne durchgeführt, die typischerweise eine Sumpfzone, eine Reaktionszone und eine Kopfzone umfasst und neben Sumpf- und Kopfaustragsströmen auch einen weiteren Abzug aufweisen kann. Für eine prinzipielle Beschreibung der Reaktivdestillation und geeigneter Vorrichtungen wird auf WO 2005/073177 verwiesen.

Das Verfahren wird kontinuierlich durchgeführt. Dies bedeutet, dass jeder der angegebenen Verfahrensschritte kontinuierlich durchgeführt wird im Unterschied zu einem diskontinuierlichen oder Batch-Verfahren. Die Ausbildung der kontinuierlichen Fahrweise entsprechender Verfahrensschritte ist dem Fachmann bekannt. Bezogen auf das Gesamtverfahren, werden die Ausgangsstoffe kontinuierlich in die Verfahrenssequenz eingetragen, und die Verfahrensprodukte werden kontinuierlich aus der Verfahrenssequenz ausgetragen, so dass das gesamte Verfahren über einen längeren Zeitraum unterbrechungsfrei kontinuierlich betrieben werden kann. Nur durch zu große Katalysator-Deaktivierungen können sich Unterbrechungen des Verfahrensablaufs ergeben, wenn der Katalysator ausgetauscht oder regeneriert werden soll oder muss. Es kann aber auch eine kontinuierliche Ausschleusung, Regenerierung oder Erneuerung des Katalysators erfolgen.

In diesem Sinne ist der anspruchsgemäße Begriff "kontinuierliche Herstellung" zu verstehen.

Anspruchsgemäß werden in den Aufarbeitungen 1 und 2 Produkte abgetrennt und teilweise in andere Verfahrensschritte zurückgeführt.

Sowohl die Abtrennung, als auch die Rückführung können jeweils vollständig oder teilweise erfolgen. Dies bedeutet, dass erfindungsgemäß auch eine teilweise Rückführung der jeweiligen Ströme in andere Verfahrensschritte umfasst werden soll, wie auch die nur teilweise Abtrennung einzelner Komponenten bei der Aufarbeitung.

Vorzugsweise erfolgt die Abtrennung und Rückführung im Wesentlichen vollständig oder vollständig.

Ferner ist angegeben, dass die Reaktionsausträge oder Destillationsausträge bestimmte Komponenten enthalten. Es ist erfindungsgemäß auch möglich und bevorzugt, dass diese Gemische im Wesentlichen oder vollständig aus den angegebenen Komponenten bestehen und damit keine wesentlichen Mengen weiterer chemischer Verbindungen oder keine weiteren chemischen Verbindungen enthalten.

Der erfindungsgemäß verwendete Begriff "enthaltend" oder "enthalten" kann damit bevorzugt auch "im Wesentlichen bestehen aus" oder "besteht im Wesentlichen aus" oder "bestehen" oder "besteht aus" bedeuten.

Unter 3-Pentennitril werden im Rahmen der vorliegenden Anmeldung Gemische aus den hydrocyanierbaren Pentennitril-Isomeren trans-3-Pentennitril, cis-3-Pentennitril und 4-Pentennitril verstanden. Die vorstehende Tabelle zeigt, dass alle drei Isomere bei Normaldruck zwischen 140 und 147°C sieden.

Durch die erfindungsgemäße Verfahrensweise kann das Gesamtverfahren und insbesondere die Isomerisierung über einen langen Zeitraum kontinuierlich betrieben werden. Selbst bei kontinuierlicher Isomerisierung in Schritt b) bei der bevorzugten Temperatur von 150 bis 220°C wird keine störende Oligomerenbildung beobachtet, die den Katalysator desaktivieren würde.

Es war zu erwarten, dass eine Oligomerenbildung von 1,4% bei 126 bis 144°C im diskontinuierlichen Versuch zur Verkürzung der Katalysator-Standzeit bei kontinuierlicher Fahrweise (150 bis 220°C) und zu merklichen Ausbeute-Verlusten führen würde.

Die einzelnen Verfahrensschritte werden nachstehend näher erläutert.

Die vorliegende Erfindung betrifft ein kontinuierliches Verfahren zur Herstellung von Adipodinitril durch Hydrocyanierung von 3-Pentennitril mit Blausäure in Gegenwart von Nickel(0)-Phosphorligand-Komplexen, die bevorzugt bidentate oder polydentate, insbesondere bidentate Phosphine, Phosphonite, Phosphinite oder Phosphite als Liganden enthalten. Besonders bevorzugt sind bidentate Phosphite. In dieses Hydrocyanierungsverfahren wird die Isomerisierung des Nebenprodukts cis-2-Pentennitril zu 3-Pentennitril und dessen Rückführung integriert:
Bei der 3-Pentennitril-Hydrocyanierung entstehen neben dem Zielprodukt Adipodinitrilverzweigte Dinitrile wie 2-Methylglutardinitril, die nicht hydrocyanierbaren Pentennitrile cis-und trans-2-Pentennitril und die Methylbutennitrile cis- und trans-2-Methyl-2-butennitril. In Aufarbeitung 1 werden Roh-Adipodinitril und Lewis-Säure augeschleust. Nickel(0)-Katalysator wird durch Extraktion abgetrennt und - gegebenenfalls nach Regenerierung - in die 3-Pentennitril-Hydrocyanierung zurückgeführt.

Cis-2-Pentennitril lässt sich in Aufarbeitung 1 zusammen mit cis-2-Methyl-2-butennitril von den restlichen linearen Pentennitrilen trans-2-Pentennitril, nicht umgesetztem cis- und trans-3-Pentennitril und 4-Pentennitril destillativ abtrennen und kontinuierlich zu 3-Pentennitril enthaltenden Isomerengemischen isomerisieren. Gemische aus trans-2-Pentennitril, den 3-Pentennitrilen und 4-Pentennitril werden nach Abtrennung von trans-2-Methyl-2-butennitril in die 3-Pentennitril-Hydrocyanierung zurückgeführt.

Der Reaktionsaustrag der kontinuierlichen cis-2-Pentennitril-Isomerisierung wird in Aufarbeitung 2 destillativ aufgearbeitet. Cis-2-Pentennitril wird in die Isomerisierung zurückgeführt, cis-2-Methyl-2-butennitril ausgeschleust und ein Gemisch aus trans-2-Pentennitril und 3-Pentennitril in die 3-Pentennitril-Hydrocyanierung zurückgeführt.

Eine Ausführungsform des erfindungsgemäßen kontinuierlichen Verfahrens zur Herstellung von Adipodinitril aus 3-Pentennitril und Blausäure in Gegenwart von Nickel(0)-Phosphorligand-Komplexen als Katalysatoren unter Einbeziehung der Isomerisierung von cis-2-Pentennitril zu 3-Pentennitril ist in Figur 1 schematisch dargestellt.

Die in Figur 1 verwendeten Abkürzungen bedeuten dabei
- PN:: Pentennitril
- 2M2BN:: 2-Methyl-2-butennitril
- Isom:: Isomerisierung
- Auf:: Aufarbeitung
- Hydro:: Hydrocyanierung
- R1: der Reaktor 1
- R2: der Reaktor 2
- Kat:: Katalysator
- Lewis-S:: Lewis-Säure
- Kat-Reg:: Katalysator-Regenerierung
- ADN:: Roh-Adipodinitril

Figur 2 zeigt schematisch die Aufarbeitungen 1 und 2 und dabei ist die Bedeutung der Abkürzungen wie vorstehend angegeben.

Die bevorzugten Verfahrensschritte werden nachfolgend näher erläutert. Dabei können die einzelnen Schritte oder mehrere der Schritte auf die 4 erfindungsgemäßen Verfahrensvarianten angewendet werden.

### Verfahrensschritt (a) (Hydrocyanierung von 3-Pentennitril)

Verfahrensschritt a) umfasst die Hydrocyanierung von 3-Pentennitril mit Blausäure an mindestens einem Nickel(0)-Phosphorligand-Komplex als Katalysator in Gegenwart von freiem Liganden und mindestens einer Lewis-Säure. Die Hydrocyanierung von 3-Pentennitril mit Blausäure zu Adipodinitril kann in an sich bekannter Weise, z. B. gemäß WO 2005/073167 und insbesondere WO 2005/073172 durchgeführt werden.

Beispielsweise können die in diesen Anmeldungen genannten, Phosphor enthaltenden bidentaten und polydentaten Liganden, die mit ihnen hergestellten Nickel(0)-Phosphorligand-Komplexe und die genannten Lewis-Säuren erfindungsgemäß verwendet werden.

Besonders bevorzugt sind bidentate und polydentate Liganden aus der Gruppe der Phosphite, der Phosphinite und der Phosphonite. Ganz besonders bevorzugt sind bidentate Phosphite.

Die Verwendung von bidentaten und polydentaten, Phosphor enthaltenden Liganden in den Nickel(0)-Katalysatorkomplexen macht eine technisch praktikable cis-2-Pentennitril-Isomerisierung besonders vorteilhaft.

Bei Verwendung von monodentaten Phosphor-Liganden können gegebenenfalls die NickelKomplexe durch die Anwesenheit von 2-Pentennitrilen desaktiviert werden, da die bei der cis-2-Pentennitril-Isomerisierung auftretende Menge an cis- und trans-2-Pentennitril (bezogen auf die Summe aller Pentennitrile) oberhalb von 5% liegen kann, siehe US 3,564,040.

Bevorzugte Lewis-Säuren sind Zinkchlorid, Eisenchlorid und Triphenylbor.

Der Hydrocyanierungsaustrag aus Reaktor R1 (Strom 1) enthält im Wesentlichen das Zielprodukt Adipodinitril, weiterhin 2-Methylglutaronitril, nicht umgesetztes 3-Pentennitril, cis-2-Pentennitril, trans-2-Pentennitril, cis-und trans-2-Methyl-2-butennitril, bidentate oder polydentate Nickel(0)-Phosphorligand-Komplexe, die zugehörigen freien, Phosphor enthaltenden Liganden, ihre Abbauprodukte und mindestens eine Lewis-Säure.

Der Verfahrensschritt (a) des erfindungsgemäßen Verfahrens wird vorzugsweise bei einem absoluten Druck von 0,1 bis 10 bar, besonders bevorzugt 0,5 bis 2 bar, insbesondere 0,8 bis 1,5 bar, durchgeführt. Die Temperatur beträgt in Verfahrensschritt (a) vorzugsweise 40 bis 150°C, besonders bevorzugt 50 bis 100°C, insbesondere 60 bis 70°C.

Der Verfahrensschritt (a) kann in jeder geeigneten, dem Fachmann bekannten Vorrichtung durchgeführt werden. Für die Reaktion kommen hierfür übliche Apparaturen in Betracht, wie sie beispielsweise in Kirk-Othmer, Encyclopedia of Chemical Technology, 4.Edition, Volume 20, John Wiley and Sons, New York 1996, Seiten 1040 bis 1055, beschrieben sind. Beispiele sind Rührkesselreaktoren, Schlaufenreaktoren, Gasumlaufreaktoren, Blasensäulenreaktoren oder Rohrreaktoren, jeweils gegebenenfalls mit Vorrichtungen zur Abfuhr von Reaktionswärme. Die Reaktion kann in mehreren, wie zwei oder drei Reaktoren, durchgeführt werden.

Erfindungsgemäß werden vorteilhafte Destillationskonzepte zur Abtrennung der Nebenprodukte cis-und trans-2-Methyl-2-butennitril verwirklicht.

In den Aufarbeitungen 1 und 2 werden zwei Destillationsvorrichtungen mit Seitenabzügen verwendet. Hierdurch wird die notwendige Zahl an Destillationsvorrichtungen verringert.

### Verfahrensschritt (b) (Aufarbeitung1)

Aus dem Hydrocyanierungs-Austrag (Strom 1) kann zunächst ein Teil der nicht umgesetzten Pentennitrile als Kopfprodukt destillativ abgetrennt werden. Dies geschieht bevorzugt, falls bei der nachfolgenden Extraktion der Nickel(0)-Katalysatoren und der freien Liganden mit Kohlenwasserstoffen als Extraktionsmittel keine Phasentrennung erfolgt.

Das Sumpfprodukt der teilweisen Pentennitril-Abtrennung enthält den mindestens einen Nickel(0)-Katalysator, Katalysator-Abbauprodukte, freien Liganden, die mindestens eine Lewis-Säure, Adipodinitril und Methylglutaronitril. Aus diesem Gemisch werden der Nickel(0)-Phosphorligand-Komplex und überschüssige, Phosphor enthaltende Liganden durch Extraktion mit Kohlenwasserstoffen abgetrennt. Die Nickel(0)-Komplexe(Strom 4) werden ganz oder teilweise in eine der beiden Hydrocyanierungsstufen zurückgeführt oder zunächst ganz oder teilweise regeneriert und dann erst zurückgeführt (siehe Abbildung 1/1 in WO 2005/073172).

Insbesondere bei Verwendung von Nickel(0)-Katalysator-Komplexen, die bidentate oder polydentate Phosphorliganden enthalten (siehe z.B.US 2009/0182164A1), werden im Allgemeinen Adipodinitril-Ausbeuten von über 90% erzielt. Damit sinkt die Menge an nicht umgesetztem 3-Pentennitril auf unter 10%. Daher erübrigt sich meist eine der Katalysator-Extraktion vorgelagerte Pentennitril-Abtrennung. Diese bevorzugte Variante ist in Figur 1 schematisch dargestellt (die Katalysator-Extraktion ist in Figur 1 weggelassen).

Der von Nickel(0)-Katalysator und phosphorhaltigen Liganden befreite Hydrocyanierungsaustrag wird destillativ aufgearbeitet, beispielsweise wie in WO 2005/073172, Seiten 14 bis 16, beschrieben. Er enthält im Wesentlichen cis-2-Methy-2-butennitril, cis-2-Pentennitril, trans-2-Methyl-2-butennitril, trans-2-Pentennitril, cis-3-Pentennitril, trans-3-Pentennitril, 4-Pentennitril, Adipodinitril, Methylglutaronitril und Katalysator-Abbauprodukte.

Die destillative Auftrennung dieses Gemisches kann in jeder geeigneten, dem Fachmann bekannten Apparatur durchgeführt werden. Die Destillation findet vorzugsweise in mindestens zwei Destillationskolonnen statt. Als Einbauten für die Destillationskolonnen werden vorzugsweise strukturierte Blechpackungen, strukturierte Gewebepackungen, Glockenböden, Dualflow-Böden oder Schüttungen aus Füllkörpern und deren Kombinationen als trennwirksame Einbauten verwendet. Die Destillationskolonnen können mit einem oder mehreren flüssigen oder gasförmigen Seitenabzügen ausgeführt sein. Destillationskolonnen können als Trennwandkolonne mit einem oder mehreren vorhandenen gasförmigen oder flüssigen Seitenabzügen ausgeführt werden.

In einer ersten Destillationskolonne mit trennwirksamen Einbauten wird als Kopfprodukt ein Gemisch aus cis-2-Pentennitril und cis-2-Methyl-2-butennitril (Strom 2), aus einem Seitenabzug ein Gemisch aus trans-2-Methyl-2-butennitril, trans-2-Pentennitril, cis-3-Pentennitril, trans-3-Pentennitril und 4-Pentennitril, und als Sumpfprodukt ein Gemisch aus Adipodinitril, Methylglutaronitril, Lewis-Säure und Katalysator-Abbauprodukten (Strom 5) abgezogen.

Das als Kopfprodukt gewonnene Gemisch aus cis-2-Pentennitril und cis-2-Methyl-2-butennitril (Strom 2) kann als Ausgangsprodukt für die kontinuierliche Isomerisierung von cis-2-Pentennitril verwendet werden.

Es ist aber auch möglich, die beiden Verbindungen vor der Isomerisierung destillativ voneinander zu trennen, im Wesentlichen reines cis-2-Pentennitril für die Isomerisierung einzusetzen und das abgetrennte cis-2-Methyl-2-butennitril, das möglichst wenig cis-2-Pentennitril enthält, aus dem Verfahren auszuschleusen.

Das aus dem Seitenabzug der ersten Kolonne erhaltene Gemisch wird in einer zweiten Kolonne so aufgetrennt, dass als Kopfprodukt trans-2-Methyl-2-butennitril (Strom 6) ausgeschleust werden kann. Als Sumpfprodukt wird ein Gemisch aus trans-2-Pentennitril, cis-3-Pentennitril, trans-3-Pentennitril und 4-Pentennitril (Strom 3) erhalten, das in den Hydrocyanierungsreaktor R1 zurückgeführt wird.

Das Sumpfprodukt der ersten Kolonne(Strom 5), das Adipodinitril, 2-Methylglutaronitril, Lewis-Säure und Katalysator-Abbauprodukte enthält (Roh-Adipodinitril), wird in weiteren Aufarbeitungsschritten zu reinem, als Faservorprodukt geeignetem Adipodinitril aufgearbeitet.

### Verfahrensschritt c) (cis 2-Pentennitril-Isomerisierung)

In Verfahrensschritt c) wird das in der Aufarbeitung 1 abgetrennte Gemisch aus cis-2-Pentennitril und cis-2-Methyl-2-butennitril (Strom 2) oder im Wesentlichen reines cis-2-Pentennitril in einen Reaktor R2 geleitet und dort in Gegenwart eines heterogenen, fest angeordneten Aluminiumoxid-Katalysators zu 3-Pentennitril enthaltenden Pentennitril-Gemischen isomerisiert.

Als Katalysatoren werden Aluminiumoxide verwendet, die zusätzlich Alkali- und Erdalkalioxide oder Hydroxide enthalten können.

Aluminiumoxid kann in unterschiedlichen Modifikationen oder Gemischen verschiedener Modifikationen eingesetzt werden. Besonders bevorzugt ist Al₂O₃, das in Form von beta-, gamma-, chi-, kappa-, delta-, theta- und eta-Al₂O₃ vorliegt (M. Beller, A. Renken und R. van Santen, Catalysis, From Principles to Applications, Wiley VCH, 2012, Seiten 436-438). Diese Vielfalt von Modifikationen entsteht beim Erhitzen von Aluminiumhydroxiden auf unterschiedliche Temperaturen (Römpp, Lexikon der Chemie, 10. Auflage 1996, Stichwort Aluminiumoxid, Seite 142). Dabei bilden sich bevorzugt Mischformen der verschiedenen Modifikationen. Dies dürfte der Grund dafür sein, dass Al₂O₃, z. B. auf Firmen-Merkblättern kaum durch die jeweilige Modifikation charakterisiert wird.

Ullmanns Encyclopedia of Industrial Chemistry, 6.Auflage, Band 2, Seiten 371 bis 378, Wiley VCH, 2003, Seite 273, 6.2 gibt an, dass Al₂O₃ vor allem in Form von "aktiviertem Al₂O₃" als Katalysator verwendet wird.

Al₂O₃-Katalysatoren sind erfindungsgemäß dann für die kontinuierliche Isomerisierung von cis-2-Pentennitril zu 3-Pentennitril geeignet, wenn sie eine BET-Oberfläche von 50 bis 450 m²/g, bevorzugt 100 bis 420 m²/g, besonders bevorzugt 100 bis 400 m²/g besitzen. Je höher die BET-Oberfläche, desto schneller die Isomerisierung und desto höher die Raum-Zeit-Ausbeute.

Unter der BET-Oberfläche wird im Sinne der vorliegenden Erfindung die spezifische Oberfläche verstanden, die durch Messung der physisorbierten Stickstoff-Gasmenge nach dem in Brunauer, Ernmett, Teller, J. am. Chem. Soc., 60, (1938), Seite 309, beschriebenen Verfahren bestimmt wird.

Für die kontinuierliche Isomerisierung von cis-2-Pentennitril zu 3-Pentennitril enthaltenden Pentennitril-Gemischen sind grundsätzlich saure, neutrale oder basische Aluminiumoxide geeignet. Die Bestimmung der Acidität, Neutralität oder Basizität erfolgt durch Messung des pH-Werts. Hierbei werden die pH-Werte von 10 gew.%igen Suspensionen des jeweiligen Aluminiumoxids in Wasser bei Raumtemperatur (25°C) an einer pH-Elektrode gemessen.

Für die erfindungsgemäße kontinuierliche Isomerisierung von cis-2-Pentennitrlil zu 3-Pentennitril enthaltende Pentennitril-Gemische können saure, neutrale oder basische Aluminiumoxide mit pH-Werten von 4 bis 10,5, bevorzugt 7 bis 10,5 verwendet werden.

Beispiele geeigneter Katalysatoren sind

| Al₂O₃-Bezeichnung | Firma | pH-Wert | BET-Oberfläche [m²/g] |
|---|---|---|---|
| WA-1 Prod.Nr. 199966 | Sigma-Aldrich | 4,5 ± 0,5¹⁾ sauer | 155 |
| WN-3 Prod.Nr. 199974 | Sigma-Aldrich | 7,0 ± 0,5¹⁾ neutral | 155 |
| W B-2 Prod.Nr. 199443 | Sigma-Aldrich | 9,5 ± 0,5¹⁾ basisch | 155 |
| F-200 | BASF SE | 9,6 - 9,7²⁾ basisch | 350 |
| AC 108-1000 | Nanoscale | 7,9-8,1²⁾ basisch | 311 |
| Pural^{®}Mg30 MgO-Al₂O₃ | Sasol | 9,7 | |
| 58A | Sigma-Aldrich | 6 ± 0,5 | 150 |
| WN-6 | Sigma-Aldrich | 7,3-8,0 basisch | 200 |

| | | | |
|---|---|---|---|
| 1) aus Datenblatt 2) eigene Messungen | | | |

So erreicht man mit dem sauren Al₂O₃-Katalysator WA-1 (pH-Wert 4,5) nach sieben Stunden Reaktionszeit nur 10,1% 3-Pentennitril. Andererseits ist die Oligomerenbildung mit 0,01% gering.

Der neutrale Al₂O₃-Katalysator WN-3 (pH 7) erreicht nach 7 Stunden Reaktionszeit mit 22,7% 3-Pentennitril fast die Werte des basischen Katalysators F 200 (23,9%), isomerisiert jedoch, wie die 3-Pentennitril-Werte nach 2 Stunden (14,8% gegenüber 22,9 %) zeigen, langsamer. Die Oligomeren-Menge steigt auf 0,29 % an.

Der basische Katalysator F- 200 (pH 9,6-9,7) isomerisiert cis-2-Pentennitril deutlich schneller und erreicht mit 23,9 % die höchsten 3-Pentennitril-Werte. Dagegen steht der starke Anstieg des Oligomerenanteils auf 1,45%.

Die erfindungsgemäßen Al₂O₃-Katalysatoren werden vorzugsweise als Formkörper verwendet. Unter Formkörpern werden erfindungsgemäß nicht Split oder Pulver verstanden, sondern durch Formgebungsprozesse mehr oder weniger einheitlich gestaltete Formkörper, wie Stränge, Tabletten, Zylinder, Monolithen. Die Formkörper können dabei beliebige dreidimensionale Gestalt annehmen.

Das Aluminiumoxid kann in reiner Form vorliegen.

Dabei können 10 bis 100 Gew.-% Aluminiumoxide aus Boehmit aufgebaut sein.

Es ist zudem möglich, Aluminiumoxid einzusetzen, das weitere Verbindungen enthält, wie Seltenerdenoxide, beispielsweise Ceroxid, Lanthanoxid, Praseodymoxid, Alkalioxide, Erdalkalioxide oder deren Gemische. Solche Verbindungen können in Mengen von mindestens 10 Gew.-ppm bis höchstens 30 Gew.-%, vorzugsweise höchstens 20 Gew.-%, insbesondere höchstens 10 Gew.-%, bezogen auf die Summe aus Aluminiumoxid und solchen Verbindungen, enthalten sein.

Weiterhin können neben dem Oxid-Anion weitere Anionen, wie Hydroxy-Anionen, vorliegen.

Das Gesamt-Porenvolumen der Al₂O₃-Katalysatoren mit einer BET-Oberfläche von 50 bis 450 m²/g beträgt vorzugsweise 0,5 bis 1 cm³/g, bevorzugt 0,6 bis 0,95 cm³/g.

Die Katalysator-Belastung beträgt vorzugsweise 0,05 bis 50, besonders bevorzugt 0,1 bis 10, insbesondere 0,2 bis 5 kg cis-2-Pentennitril pro Liter Katalysator und Stunde.

Die mittlere Verweilzeit am Katalysator liegt vorzugsweise bei 0,05 bis 10 Stunden, besonders bevorzugt 0,1 bis 8 Stunden, insbesondere 0,25 bis 1 Stunde.

Die Isomerisierungstemperatur beträgt 120 bis 220°C, vorzugsweise 150 bis 220°C, bevorzugt 160 bis 210°C, besonders bevorzugt 170 bis 200°C. Der Isomerisierungsdruck beträgt 1 bis 15 bar, vorzugsweise 2 bis 5 bar, bevorzugt 3 bis 4 bar. Unter Druck wird vorzugsweise gearbeitet, um das Reaktionsgemisch flüssig zu halten.

Die kontinuierliche Isomerisierung von cis-2-Pentennitril kann in Gegenwart eines unter den Reaktionsbedingungen inerten Lösungsmittels durchgeführt werden. Hierfür kommen z. B. Kohlenwasserstoffe wie Cyclohexan oder n-Hexan oder Ether wie z. B. Tetrahydrofuran oder Dioxan in Frage. Die auf cis-2-Pentennitril bezogene Lösungsmittel-Menge beträgt 5 bis 50 Gew.-%, bevorzugt 10 bis 40 Gew.-%, besonders bevorzugt 20 bis 30 Gew.-%. Bevorzugt wird in Abwesenheit eines Lösungsmittels gearbeitet.

Die kontinuierliche Isomerisierung von cis-2-Pentennitril kann in dem Fachmann bekannten Apparaturen durchgeführt werden.

Als Reaktoren können prinzipiell alle Reaktoren eingesetzt werden, die für die Durchführung heterogen katalysierter Reaktionen in der Flüssigphase unter Druck geeignet sind. Hierzu gehören Reaktoren zur Suspensionsfahrweise, zur Wirbelschichtfahrweise und bevorzugt zur Festbettfahrweise. Geeignete Reaktoren sind dabei Rührkessel, Wirbelschichtreaktoren, Strahlschlaufenreaktoren, Strahldüsenreaktoren, Blasensäulenreaktoren und bevorzugt Rohrreaktoren. Dabei kann die Isomerisierung in Sumpf- oder Rieselfahrweise erfolgen.

Bei der Isomerisierung eines Gemischs aus cis-2-Methyl-2-butennitril und cis-2-Pentennitril wird überraschenderweise nur cis-2-Pentennitril isomerisiert.

Zu erwarten war, dass auch cis-2-Methyl-2-butennitril isomerisiert wird. Dabei würde 2-Methyl-3-butennitril entstehen, das die Aufarbeitung aufgrund seines Siedepunktes von 124°C/1013 mbar erschwert hätte. Dies tritt nicht ein. Daher kann ein Gemisch aus cis-2-Pentennitril und cis-2-Methyl-2-butennitril isomerisiert werden.

Es ist zudem bekannt, dass Acrylnitril leicht polymerisiert (Beyer/Walter, Lehrbuch der Organischen Chemie, 24. Auflage, Seite 254, S. Hirzel Verlag Stuttgart/Leipzig).

Cis- und trans-2-Pentennitril stellen isomere 3-Ethylacrylnitrile dar. Die bei 126 bis 144°C diskontinuierlich durchgeführten Versuche zur Isomerisierung von cis-2-Pentennitril in Gegenwart von Aluminiumoxiden als Katalysator zeigen, dass Oligomere (Dimere) gebildet werden (siehe die nachfolgenden Beispiele). Dabei steigt die Oligomeren-Menge mit zunehmender BET-Oberfläche und zunehmender Basizität der Aluminiumoxide an.

Es war nicht vorauszusehen, ob bei der erfindungsgemäßen Isomerisierung bei bevorzugt 150 bis 220°C eine deutlich stärkere Oligomerenbildung stattfinden würde. Eine schnelle Desaktivierung des Katalysators durch dessen Belegung mit Oligomeren, eine Verstopfung der Isomerisierungs-Apparatur und/oder ein deutlicher Verlust an Pentennitrilen könnten die Folge sein.

Überraschenderweise wurde im kontinuierlichen Versuch eine hohe Katalysator-Standzeit von über 3000 Stunden und eine tolerable Oligomerenbildung beobachtet.

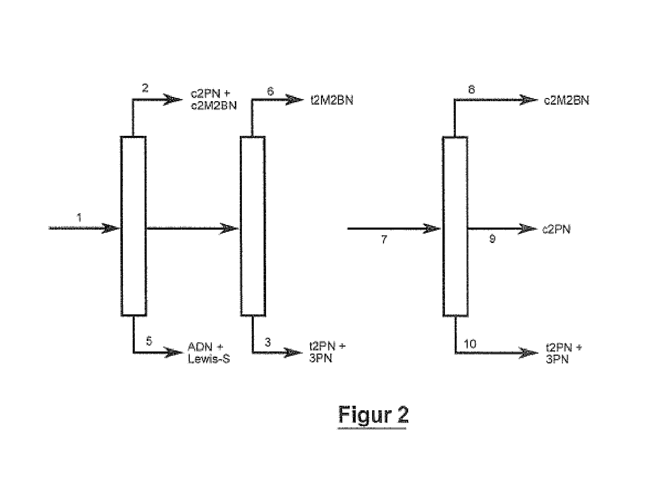

Verfahrensschritt (d) (Aufarbeitung 2, siehe Figur 1 und Figur 2)

Der Reaktionsaustrag aus der kontinuierlichen Isomerisierung von cis-2-Pentennitril (Strom 7) enthält im Wesentlichen nicht umgesetztes cis-2-Pentennitril, trans-2-Pentennitril, cis-3-Pentennitril, trans-3-Pentennitril, 4-Pentennitril und, sofern nicht schon in Aufarbeitung 1 abgetrennt, auch cis-2-Methyl-2-butennitril.

Falls cis-2-Methyl-2-butennitril noch in Strom 7 enthalten ist, wird es in einer Destillationsvorrichtung als Kopfprodukt von cis-2-Pentennitril abgetrennt (Strom 8) und ausgeschleust.

Aus einem Seitenabzug wird gegebenenfalls nicht umgesetztes cis-2-Pentennitril entnommen und in die kontinuierliche Isomerisierung R2 zurückgeführt (Strom 9).

Das Sumpfprodukt der Destillationsvorrichtung (Strom 10) enthält im Wesentlichen trans-2-Pentennitril, cis-3-Pentennitril, trans-3-Pentennitril und 4-Pentennitril.

Strom 3 wird ebenso wie Strom 10 in die 3-Pentennitril-Hydrocanierung zurückgeführt. Dabei können beide Ströme einzeln oder nach Vereinigung zurückgeführt werden.

Alternativer Verfahrensschritt c) (Reaktivdestillation von cis-2-Pentennitril)

In einem bevorzugten Verfahrensschritt c) wird die kontinuierliche Isomerisierung von cis-2-Pentennitril zu 3-Pentennitril (Verfahrensschritt c)) in Form einer Reaktivdestillation durchgeführt. Eine Reaktivdestillation kann die vorstehenden Schritte c) und d) zusammenfassen. Reaktivdestillation von cis-2-Pentennitril zu 3-Pentennitril bedeutet, dass die kontinuierliche Isomerisierung von cis-2-Pentennitril zu 3-Pentennitril und die destillative Aufarbeitung des Isomerisierungs-Austrags in einem Apparat vereinigt ist, in dem die Isomerisierung und die destillative Aufarbeitung des Isomerisierungs-Gemischs stattfinden. In Figur 1 werden dann der Isomerisierungsreaktor R2 und die Aufarbeitung 2 zu einem den Katalysator enthaltenden Apparat vereinigt, aus dem bei vollständigem cis-2-Pentennitril-Umsatz und bei vorheriger Abtrennung von 2-Methyl-2-butennitril ein in die 3-Pentennitril-Hydrocyanierung rückführbarer Strom aus trans-2-Pentennitril, cis- und trans-3-Pentennitril und 4-Pentennitril entnommen werden kann.

Die Isomerisierung von cis-2-Pentennitril zu 3-Pentennitril in Gegenwart von Aluminiumoxid als Katalysator ist in WO 2005/073177A1 als Reaktivdestillation beschrieben. Es kann apparativ wie dort beschrieben vorgegangen werden.

Für die erfindungsgemäße Reaktivdestillation gelten die in der vorliegenden Anmeldung beschriebenen Reaktionsbedingungen der kontinuierlichen cis-2-Pentennitril-Isomerisierung wie Temperatur, Druck, Verweilzeit und Stoffeigenschaften wie BET-Oberfläche, Porenvolumen und pH-Werte des Al₂O₃.

Die Erfindung wird durch die nachfolgenden Beispiele näher erläutert.

### Ausführungsbeispiele

### Diskontinuierliche Isomerisierung von cis-2-Pentennitril (orientierende Versuche, nicht erfindungsgemäß)

Die diskontinuierliche Isomerisierung von cis-2-Pentennitril wurde in einem 250 ml Mehrhalskolben mit Rührer, Thermometer, Kühler und Septum zur Probennahme durchgeführt. Pro Versuch wurden 120 g cis-2-Pentennitril der Firma Merck (CAS 25899-50-7) in dem Kolben vorgelegt.

Das cis-2-Pentennitril wurde mit 10 Gew.-% Al₂O₃-Pulver (im Mörser zu Pulver zerrieben) gemäß nachstehender Tabelle versetzt und bei Normaldruck 7 Stunden lang zum Rückfluss (126 bis 144°C) erhitzt. Die Temperatur erhöhte sich im Verlauf der Isomerisierung durch die Bildung von höher als cis-2-Pentennitril siedenden Pentennitrilen.

Nach 15 und 30 Minuten, dann jeweils nach einer Stunde, wurden Proben gezogen und gaschromatographisch auf ihren Gehalt an Pentennitrilen untersucht.

Als GC-Säule wurde eine CP-Wax52CB-Trennsäule verwendet. Das GC-Temperaturprogramm betrug 5 Minuten isotherm bei 50°C, dann 8°C Temperatur-Steigerung pro Minute bis zu einer Endtemperatur von 240°C.

Die pH-Werte der Aluminiumoxide stammen entweder aus den Merkblättern der Herstellfirmen oder wurden gemessen.

Für pH-Messungen im Labor wurden 5 g Katalysator-Pulver mit 45 g Wasser in ein Becherglas gegeben und die resultierende 10 gew.-%ige Suspension mit einem Rührer (5000 Umdrehungen pro Minute) bei Raumtemperatur gerührt. Der pH-Wert wurde mit einer kalibrierten pH-Elektrode (Blue Line 18pH, SI. Analysis) zu verschiedenen Zeiten (z.B.nach 5 und 30 Minuten) gemessen, bis sich ein konstanter Wert ergab.

In der Tabelle sind diskontinuierliche Isomerisierungsergebnisse in Gegenwart von Aluminiumoxiden als Katalysatoren in Abhängigkeit von steigender BET-Oberfläche und steigendem pH -Wert zusammengestellt. Die Ergebnisse zeigen, dass die Geschwindigkeit der Isomerisierung mit zunehmender BET-Oberfläche und steigendem pH-Wert ansteigt. Gleichzeitig werden jedoch zunehmende Mengen an Oligomeren (einschließlich Dimeren) beobachtet.

| Beispiel Nr. | Al₂O₃-Bezeichnung | Firma | pH-Wert | BET-Oberfläche [m²/g] | Reaktionszeit [h] | c-2 PN | t-2 PN | c - + t - 3 PN + 4 PN | Oligomere |
|---|---|---|---|---|---|---|---|---|---|
| V1 | alpha-Al₂O₃ | NorPro | | 1 | 2 | 99,1 | 0 | 0,4 | 0 |
| | | | | | 7 | | | | |
| 2 | 58A | Sigma-Aldrich | 6 ± 0,5 sauer | 150 | 7 | 59,5 | 19,6 | 19,8 | 0,13 |
| 3 | WN-3 Prod.Nr. 199974 | Sigma-Aldrich | 7,0 ± 0,5¹⁾ neutral | 155 | 7 | 46,5 | 29,6 | 22,7 | 0,29 |
| 4 | WB-2 Prod.Nr. 199443 | Sigma-Aldrich | 9,5 ± 0,5¹⁾ basisch | 155 | 7 | 40,8 | 34,8 | 23,2 | 0,35 |
| 5 | WN-6 | Sigma-Aldrich | 7,3 - 8,0 basisch | 200 | 7 | 42,9 | 32,5 | 23,4 | 0,31 |
| 6 | F-200 | BASF SE | 9,6 - 9,7²⁾ basisch | 350 | 7 | 39,6 | 34,2 | 23,9 | 1,45 |
| 7 | AC 108-1000 | Nanoscale | 7,9 - 8,1²⁾ basisch | 311 | 2 | 33,6 | 38,1 | 23,6 | 3,7 |
| | | | | | 7 | 32,2 | 38,0 | 23,2 | 5,7 |
| 8 | Pural Mg 30 MgO-Al₂O₃ | Sasol | 9,7 basisch | | 2 | 37,7 | 30,5 | 23,4 | 7,6 |
| | | | | | 7 | 34,3 | 33,5 | 22,8 | 8,5 |

| | | | | | | | | | |
|---|---|---|---|---|---|---|---|---|---|
| 1) aus Datenblatt 2) eigene Messungen | | | | | | | | | |

Durch GC-MS-Kopplung wurde nachgewiesen, dass es sich bei den Hochsiedern im Bereich zwischen den Retentionszeiten 22,5 und 28 Minuten um Pentennitril-Dimere mit einem Molekulargewicht von 162 handelt.

Der Oligomerengehalt der Reaktionsausträge wird in Flächenprozenten angegeben: (Summe der Dimeren-Flächen dividiert durch die Summe aller Flächen) x 100.

Die qualitative Zusammensetzung von Aluminiumoxiden wurde durch XRD-Analyse ermittelt:
Die Aluminiumoxide 58A, WA-1, WN-3, WN-6 und WB-2 besitzen gleiche XRD-Spektren. Die Kristallinität ist gering. Als Phasen wurden gamma-Al₂O₃ (tetragonal) und chi-Al₂O₃ (kubisch) gefunden.

Der Al₂O₃-Katalysator F-200 besaß ebenfalls geringe Kristallinität. Als Hauptphase war Boehmit AIO (OH) (orthorhombisch) enthalten, weiterhin wie bei den vorangehenden Katalysatoren gamma-Al₂O₃ (tetragonal) und chi-Al₂O₃ (kubisch).

### Kontinuierliche Isomerisierung von cis-2-Pentennitril (Standzeitversuch)

Die kontinuierliche Isomerisierung von cis-2-Pentennitril zu 3-Pentennitril enthaltenden Pentennitril-Isomeren-Gemischen erfolgte in Gegenwart von Al₂O₃-Kugeln (3,2 mm Durchmesser) vom Typ Alcoa F-200, die eine BET-Oberfläche von 350 m²/g, einen pH-Wert von 9,6 bis 9,7 und ein Gesamt-Porenvolumen von 0,59 ml/g besaßen.

Das verwendete cis-2-Pentennitril besaß eine Reinheit von 98,8%, der Rest zu 100% bestand aus anderen ungesättigten isomeren C5-Nitrilen.

Die Isomerisierung erfolgte in einem 250 ml Rohrreaktor (Reaktorgeometrie 25 mm x 450 mm) in Sumpf-Fahrweise. In den Reaktor wurden 130,6 g Al₂O₃-Katalysator eingefüllt. Dies entspricht 250 ml Katalysator. Dann wurden 7 bar Argon aufgepresst und die Druckhaltung auf 10 bar eingestellt.

Beginnend bei einer Isomerisierungstemperatur von 125°C und einem Druck von 10 bar wurden pro Stunde 31,2 ml (25 g) cis-2-Pentennitril (Katalysatorbelastung 0,1 kg cis-2-Pentennitril pro Liter Katalysator und Stunde = 100 kg cis-2-Pentennitril pro m3 Katalysator und Stunde) in den Reaktor gefahren.

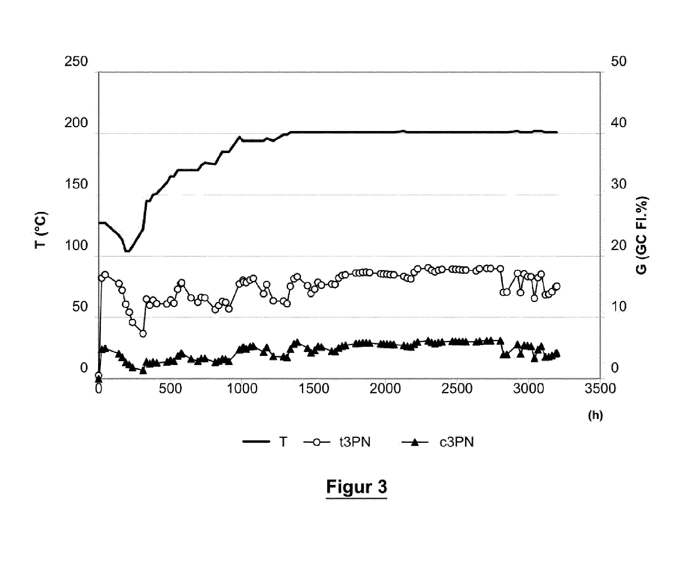

Figur 3 zeigt, dass die Temperatur zunächst bei gleichbleibender Katalysator-Belastung innerhalb von 1000 Stunden auf 200°C (Eigendruck bei 200°C etwa 4 bar) gesteigert und die Umsetzung weitere 1800 Stunden unter gleichen Reaktionsbedingungen durchgeführt wurde.

Nach 2800 Stunden wurde die Katalysator-Belastung auf 0,2 kg cis-2-Pentennitril pro Liter Katalysator und Stunde erhöht, was zu einem geänderten Kurvenverlauf führte. Der Versuch wurde nach insgesamt 3200 Stunden beendet. Der Al₂O₃-Katalysator konnte problemlos aus dem Reaktor ausgebaut werden.

In Figur 3 sind die Mengen (Gehalt G in Gaschromatogramm-Flächenprozent) an gebildetem cis- und trans-3-Pentennitril (PN) in Abhängigkeit von Temperatur (T) und Reaktionszeit (L) dargestellt. Die Graphik zeigt, dass über den gesamten Zeitraum von 3200 Stunden keine Desaktivierung des Katalysators beobachtet wurde. Verstopfungen durch Hochsieder (Oligomere) traten nicht auf.

Tabelle 3 zeigt, dass innerhalb von 1000 bis 2800 Stunden Reaktionszeit cis-+trans-3-Pentennitril-Mengen von 19,2 bis 23,9%, innerhalb von 2800 bis 3200 Stunden bei höherer Belastung Werte von 21,9 bis 19,3% erzielt wurden.

Gaschromatographisch wurden Oligomerenmengen zwischen 0,3 und 2,0 % gemessen.

Da möglicherweise nicht alle Oligomeren gaschromatographisch erfassbar waren, wurden jeweils 100 g des Isomerisierungsaustrags bei 120°C und 5 mbar über eine Destillationsbrücke destilliert. Die Destillat-Vorlage wurde gekühlt. Die Menge an nicht flüchtiger Rückstandsmenge lag durchschnittlich zwischen 1,0 und 1,5 g pro 100 g Isomerisierungsaustrag, die Destillatmengen zwischen 98,5 und 99 g. Damit wurden die gaschromatographisch ermittelten Oligomerenmengen bestätigt.

**Tabelle 3: Kontinuierliche Isomerisierung von cis-2-Pentennitril**

| Reaktionszeit [h] | c-2PN | t-2PN | c - + t-3PN + 4-PN | Oligomere | Summe FI.-% |
|---|---|---|---|---|---|
| | GC-Flächen-% | | | | |
| 501 | 69,6 | 13,4 | 15,8 | | 98,8 |
| 1006 | 50,5 | 25,7 | 21,2 | | 97,4 |
| 1506 | 56,8 | 20,2 | 19,2 | 0,3 | 96,5 |
| 2011 | 43,8 | 29,2 | 23,4 | 1,0 | 97,4 |
| 2490 | 39,6 | 31,0 | 23,9 | 2,0 | 96,5 |
| 3017 | 50,3 | 26,0 | 21,9 | 0,7 | 98,9 |
| 3196 | 61,0 | 17,0 | 19,3 | | 97,3 |

## Patentansprüche

1. Verfahren zur kontinuierlichen Herstellung von Adipodinitril durch Hydrocyanierung von 3-Pentennnitril, bei dem man
a) 3-Pentennitril oder eine Mischung enthaltend 3-Pentennitril in einem Reaktor R1 mit Blausäure in Gegenwart von mindestens einem Nickel(0)-Komplex als Katalysator, freiem Liganden und mindestens einer Lewis-Säure hydrocyaniert unter Erhalt eines Reaktionsaustrags, der Adipodinitril, 2-Methylglutaronitril, den Nickel(0)-Komplex, freien Liganden, Lewis-Säure, Katalysator-Abbauprodukte, nicht umgesetztes 3-Pentennitril und als Nebenkomponenten cis- und trans-2-Methyl-2-butennitril sowie cis- und trans-2-Pentennitril enthält,
b) in einer Aufarbeitung 1 aus dem Reaktionsaustrag des Reaktors R1 in einer ersten Destillationsvorrichtung als Kopfprodukt ein Gemisch enthaltend cis-2-Methyl-2-butennitril und cis-2-Pentennitril, als Sumpfprodukt ein Gemisch enthaltend Adipodinitril, Nickel(0)-Komplex, freien Liganden, mindestens eine Lewis-Säure und Katalyator-Abbauprodukte und an einem Seitenabzug ein Gemisch enthaltend trans-2-Methyl-2-butennitril, trans-2-Pentennitril und 3-Pentennitril abtrennt, das Seitenabzugsprodukt in einer zweiten Destillationsvorrichtung so auftrennt, dass als Kopfprodukt trans-2-Methyl-2-butennitril abgetrennt und ausgeschleust wird und als Sumpfprodukt 3-Pentennitril und trans-2-Pentennitril erhalten und in die 3-Pentennitril-Hydrocyanierung in Schritt a) zurückgeführt werden,
c) das Gemisch enthaltend cis-2-Methyl-2-butennitril und cis-2-Pentennitril aus Schritt b) in einem Reaktor R2 in Gegenwart von Aluminiumoxid als Katalysator kontinuierlich zu einem 3-Pentennitril enthaltenden Produktgemisch isomerisiert, wobei die Isomerisierung bei Temperaturen von 120 bis 220°C und Drücken von 1 bis 15 bar in der Flüssigphase erfolgt, das Aluminiumoxid eine BET-Oberfläche von 50 bis 450 m²/g und einen pH-Wert von 4 bis 10,5 besitzt,
d) aus dem Reaktionsaustrag des Reaktors R2 in einer Aufarbeitung 2 in einer Destillationsvorrichtung cis-2-Methyl-2-butennitril als Kopfprodukt abtrennt und ausschleust, nicht umgesetztes cis-2-Pentennitril aus einem Seitenabzug abtrennt und in den Reaktor R2 in Schritt c) und das 3-Pentennitril enthaltende Sumpfprodukt in die 3-Pentennitril-Hydrocyanierung in Schritt a) zurückführt.

2. Verfahren zur kontinuierlichen Herstellung von Adipodinitril durch Hydrocyanierung von 3-Pentennnitril, bei dem man
a) 3-Pentennitril oder eine Mischung enthaltend 3-Pentennitril in einem Reaktor R1 mit Blausäure in Gegenwart von mindestens einem Nickel(0)-Komplex als Katalysator, freiem Liganden und mindestens einer Lewis-Säure hydrocyaniert unter Erhalt eines Reaktionsaustrags, der Adipodinitril, 2-Methylglutaronitril, den Nickel(0)-Komplex, freien Liganden, Lewis-Säure, Katalysator-Abbauprodukte, nicht umgesetztes 3-Pentennitril und als Nebenkomponenten cis- und trans-2-Methyl-2-butennitril sowie cis- und trans-2-Pentennitril enthält,
b) in einer Aufarbeitung 1 aus dem Reaktionsaustrag des Reaktors R1 in einer ersten Destillationsvorrichtung als Kopfprodukt nur cis-2-Methyl-2-butennitril, als Sumpfprodukt alle restlichen Verbindungen abtrennt und in einer zweiten Destillationsvorrichtung das Sumpfprodukt der ersten Destillationsvorrichtung so auftrennt, dass als Kopfprodukt cis-2-Pentennitril, über einen Seitenabzug 3-Pentennitril, trans-2-Pentennitril und trans-2-Methyl-2-butennitril und als Sumpfprodukt Roh-Adipodinitril, Nickel-(0)-Komplex, freier Ligand, die mindestens eine Lewis-Säure und Katalysator-abbauprodukte erhalten werden, und das Seitenabzugsprodukt der zweiten Destillationsvorrichtung in einer dritten Destillationsvorrichtung so destilliert wird, dass trans-2-Methyl-2-butennitril als Kopfprodukt ausgeschleust wird und als Sumpfprodukt ein Gemisch enthaltend trans-2-Pentennitril und 3-Pentennitril abgetrennt und in die 3-Pentennitril-Hydrocyanierung in Schritt a) zurückgeführt wird,
c) das cis-2-Pentennitril aus Schritt b) in einem Reaktor R2 in Gegenwart von Aluminiumoxid als Katalysator kontinuierlich zu einem 3-Pentennitril enthaltenden Produktgemisch isomerisiert, wobei die Isomerisierung bei Temperaturen von 120 bis 220°C und Drücken von 1 bis 15 bar in der Flüssigphase erfolgt, das Aluminiumoxid eine BET-Oberfläche von 50 bis 450 m²/g und einen pH-Wert von 4 bis 10,5 besitzt,
d) aus dem Reaktionsaustrag des Reaktors R2 in einer Aufarbeitung 2 in einer Destillationsvorrichtung nicht umgesetztes cis-2-Pentennitril als Kopfprodukt abtrennt und in den Reaktor R2 in Schritt c) und das 3-Pentennitril enthaltende Sumpfprodukt in die 3-Pentennitril-Hydrocyanierung in Schritt a) zurückführt.

3. Verfahren zur kontinuierlichen Herstellung von Adipodinitril durch Hydrocyanierung von 3-Pentennnitril, bei dem man
a) 3-Pentennitril oder eine Mischung enthaltend 3-Pentennitril in einem Reaktor R1 mit Blausäure in Gegenwart von mindestens einem Nickel(0)-Komplex als Katalysator, freiem Liganden und mindestens einer Lewis-Säure hydrocyaniert unter Erhalt eines Reaktionsaustrags, der Adipodinitril, 2-Methylglutaronitril, den Nickel(0)-Komplex, freien Liganden, Lewis-Säure, Katalysator-Abbauprodukte, nicht umgesetztes 3-Pentennitril und als Nebenkomponenten cis- und trans-2-Methyl-2-butennitril sowie cis- und trans-2-Pentennitril enthält,
b) in einer Aufarbeitung 1 aus dem Reaktionsaustrag des Reaktors R1 in einer ersten Destillationsvorrichtung als Kopfprodukt ein Gemisch enthaltend cis-2-Methyl-2-butennitril und cis-2-Pentennitril, als Sumpfprodukt ein Gemisch enthaltend Adipodi-nitril, Nickel(0)-Komplex, freien Liganden, mindestens eine Lewis-Säure und Katalyator-Abbauprodukte und an einem Seitenabzug ein Gemisch enthaltend trans-2-Methyl-2-butennitril, trans-2-Pentennitril und 3-Pentennitril abtrennt, das Seitenabzugsprodukt in einer zweiten Destillationsvorrichtung so auftrennt, dass als Kopfprodukt trans-2-Methyl-2-butennitril abgetrennt und ausgeschleust wird und als Sumpfprodukt 3-Pentennitril und trans-2-Pentennitril erhalten und in die 3-Pentennitril-Hydrocyanierung in Schritt a) zurückgeführt werden,
c) das Gemisch enthaltend cis-2-Methyl-2-butennitril und cis-2-Pentennitril aus Schritt b) in einer Reaktivdestillationskolonne R2 in Gegenwart von Aluminiumoxid als Katalysator in einer Reaktionszone kontinuierlich zu einem 3-Pentennitril enthaltenden Produktgemisch isomerisiert, wobei die Isomerisierung bei Temperaturen von 120 bis 220°C und Drücken von 1 bis 15 bar in der Flüssigphase erfolgt, das Aluminiumoxid eine BET-Oberfläche von 50 bis 450 m²/g und einen pH-Wert von 4 bis 10,5 besitzt, und cis-2-Methyl-2-butennitril als Kopfprodukt abtrennt und ausschleust, nicht umgesetztes cis-2-Pentennitril aus einem Seitenabzug abtrennt und in den Reaktor R2 in Schritt c) und das 3-Pentennitril enthaltende Sumpfprodukt in die 3-Pentennitril-Hydrocyanierung in Schritt a) zurückführt.

4. Verfahren zur kontinuierlichen Herstellung von Adipodinitril durch Hydrocyanierung von 3-Pentennnitril, bei dem man
a) 3-Pentennitril oder eine Mischung enthaltend 3-Pentennitril in einem Reaktor R1 mit Blausäure in Gegenwart von mindestens einem Nickel(0)-Komplex als Katalysator, freiem Liganden und mindestens einer Lewis-Säure hydrocyaniert unter Erhalt eines Reaktionsaustrags, der Adipodinitril, 2-Methylglutaronitril, den Nickel(0)-Komplex, freien Liganden, Lewis-Säure, Katalysator-Abbauprodukte, nicht umgesetztes 3-Pentennitril und als Nebenkomponenten cis- und trans-2-Methyl-2-butennitril sowie cis- und trans-2-Pentennitril enthält,
b) in einer Aufarbeitung 1 aus dem Reaktionsaustrag des Reaktors R1 in einer ersten Destillationsvorrichtung als Kopfprodukt nur cis-2-Methyl-2-butennitril, als Sumpfprodukt alle restlichen Verbindungen abtrennt und in einer zweiten Destillationsvorrichtung das Sumpfprodukt der ersten Destillationsvorrichtung so auftrennt, dass als Kopfprodukt cis-2-Pentennitril, über einen Seitenabzug 3-Pentennitril, trans-2-Pentennitril und trans-2-Methyl-2-butennitril und als Sumpfprodukt Roh-Adipodinitril, Nickel-(0)-Komplex, freier Ligand, die mindestens eine Lewis-Säure und Katalysator-abbauprodukte erhalten werden, und das Seitenabzugsprodukt der zweiten Destillationsvorrichtung in einer dritten Destillationsvorrichtung so destilliert wird, dass trans-2-Methyl-2-butennitril als Kopfprodukt ausgeschleust wird und als Sumpfprodukt ein Gemisch enthaltend trans-2-Pentennitril und 3-Pentennitril abgetrennt und in die 3-Pentennitril-Hydrocyanierung in Schritt a) zurückgeführt wird,
c) cis-2-Pentennitril aus Schritt b) in einer Reaktivdestillationskolonne R2 in Gegenwart von Aluminiumoxid als Katalysator in einer Reaktionszone kontinuierlich zu einem 3-Pentennitril enthaltenden Produktgemisch isomerisiert, wobei die Isomerisierung bei Temperaturen von 120 bis 220°C und Drücken von 1 bis 15 bar in der Flüssigphase erfolgt, das Aluminiumoxid eine BET-Oberfläche von 50 bis 450 m²/g und einen pH-Wert von 4 bis 10,5 besitzt, und nicht umgesetztes cis-2-Pentennitril als Kopfprodukt abtrennt und in den Reaktor R2 in Schritt c) und das 3-Pentennitril enthaltende Sumpfprodukt in die 3-Pentennitril-Hydrocyanierung in Schritt a) zurückführt.

5. Verfahren nach einem der Ansprüche 1 bis 4, **dadurch gekennzeichnet, dass** in Schritt c) als Aluminiumoxid beta-, gamma-, chi-, kappa-, delta-, theta-, eta-Aluminiumoxid oder Gemische dieser Aluminiumoxide als Katalysator verwendet werden.

6. Verfahren nach einem der Ansprüche 1 bis 5, **dadurch gekennzeichnet, dass** in Schritt c) Aluminiumoxide mit einem pH-Wert von 7 bis 10,5 verwendet werden.

7. Verfahren nach einem der Ansprüche 1 bis 6, **dadurch gekennzeichnet, dass** in Schritt c) 10 bis 100 Gew.-% der Aluminiumoxide aus Boehmit aufgebaut sind.

8. Verfahren nach einem der Ansprüche 1 bis 7, **dadurch gekennzeichnet, dass** in Schritt c) das Aluminiumoxid als Formkörper eingesetzt wird.

9. Verfahren nach einem der Ansprüche 1 bis 8, **dadurch gekennzeichnet, dass** in Schritt c) das Aluminiumoxid 10 Gew.-ppm bis 30 Gew.-% weitere Verbindungen, bezogen auf die Summe aus Aluminiumoxid und solchen Verbindungen, enthält.

10. Verfahren nach einem der Ansprüche 1 bis 9, **dadurch gekennzeichnet, dass** in Schritt c) die BET-Oberfläche des Aluminiumoxids 100 bis 420 m²/g beträgt.

11. Verfahren nach einem der Ansprüche 1 bis 10, **dadurch gekennzeichnet, dass** in Schritt c) die Katalysator-Belastung 0,05 bis 50 kg cis-2-Pentennitril pro Liter Aluminiumoxid pro Stunde beträgt.

12. Verfahren nach einem der Ansprüche 1 bis 11, **dadurch gekennzeichnet, dass** in Schritt c) die cis-2-Pentennitril-Isomerisierung in einem Rohrreaktor in Sumpf- oder Rieselfahrweise erfolgt.

13. Verfahren nach einem der Ansprüche 1 bis 12, **dadurch gekennzeichnet, dass** in Schritt a) der Katalysator bidentate oder polydentate Phosphite, Phosphinite, Phosphonite und/oder Phosphine als Liganden enthält.

14. Verfahren nach einem der Ansprüche 1 bis 13, **dadurch gekennzeichnet, dass** in Schritt c) die Temperatur 150 bis 220°C beträgt.

15. Verfahren nach einem der Ansprüche 1 bis 14, **dadurch gekennzeichnet, dass** das im Verfahren eingesetzte 3-Pentennitril aus der Hydrocyanierung von Butadien stammt.
